# EUROPEAN PATENT APPLICATION

(11) **EP 1 676 843 A1**
(43) Date of publication of application: **05.07.2006**
(21) Application number: 04107075.6
(22) Date of filing: 31.12.2004
(51) Int. Cl.: C07D 401/06, C07D 401/14, A61K 31/404, A61P 29/00

(54) **Substituted indole ligands for the ORL-1 receptor**

(71) Applicant: NIKEM RESEARCH S.R.L., 20021 Baranzate di Bollate Milano (IT)
(72) Inventor: Ronzoni, Silvano, 20021 Baranzate di Bollate (IT); Gagliardi, Stefania, 20021 Baranzate di Bollate (IT)
(74) Representative: Gervasi, Gemma

(57) **Abstract**

New ligands for the ORL-1 receptor are described, useful for modulating the activity of said receptors in a patient in need thereof, and for preventing and treating illnesses dependent on the stimulation of this receptor. The new compounds conform to structural formula (I) 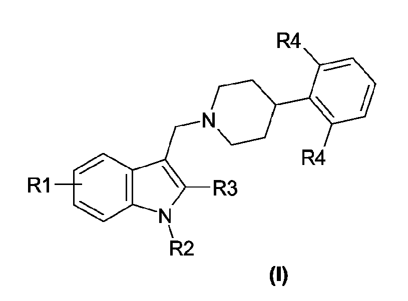 wherein R1, R2, R3, R4 are further defined in the description.

## Description

### Field of the invention

The present invention relates to certain new compounds, a process for their preparation, to pharmaceutical compositions which contain them, and to the use of these compounds in medicine. The invention relates in particular to a group of new compounds possessing antagonistic or agonistic activity for the receptors ORL-1 and useful in treating illness related to modulation of these receptors.

### Prior art

The ORL-1 receptor is located along the entire neural axis and is involved in various pathological phenomena, including the transmission of pain. Various peptide and non-peptide ligands for the ORL-1 receptor are known; the non-peptide ligands include known compounds with morphinan, benzimidazopiperidine, spiropiperidine, arylpiperidine and 4-aminoquinoline structure *(Life Sciences,* 73, 2003, 663-678). WO 0183454 and WO 03040099 describe other ORL-1 antagonists with benzosuberonylpiperidine structure substituted in position 5 by a hydroxy, alkoxy, amino or alkylamino group, and their synthesis method. *J.Med.Chem.,* 1997, 40(23), 3912-14 and WO 9709308 describe certain indoles substituted in position 3 with a dipiperazine group, as antagonists for the receptor NPY-1. *J.Med.Chem.,* 1996, 39(10), 1941-2, WO 9424105, WO 9410145, WO 02241894, WO 9629330 and GB 2076810 describe variously substituted 3-piperazinylmethyl indoles as ligands for dopamine receptors, in particular for the D4 receptor. GB 2083476 describes specific 3-arylpiperidinylmethyl indoles as 5HT uptake inhibitors. US 5215989 describes certain di-substituted piperazine and imidazole derivatives useful as class III antiarrhythmic agents. EP 846683 describes hydroxypiperidine derivatives as NMDA receptor blockers. WO 200241894 describes 2-piperazino substituted indoles, useful as antagonists for the dopamine D4 receptors. GB 1063019 describes certain piperidinoalkyl substituted indoles, useful as myorelaxants.

### Summary of the invention

It has now been found that certain substituted indoles, i.e indoles substituted in position 3 with a 4-arylpiperidinoalkyl group, are powerful ligands for the ORL-1 receptor, and can therefore be useful in the treatment and/or prevention of diseases dependent on the modulation of this receptor. They can thus be used in man or animals for treating and/or preventing pain, gastrointestinal disorders, diseases of the immune system, dysfunctions of the cardiovascular system, diseases of the excretory system, sexual dysfunction, disorders of the respiratory tract, central nervous system disorders, drug abuse, tolerance and dependence, etc. Examples of specific diseases dependent on the modulation of the ORL-1 receptor are listed further on in this specification.

### Detailed description of the invention

The compounds of the invention conform to structural formula (I) 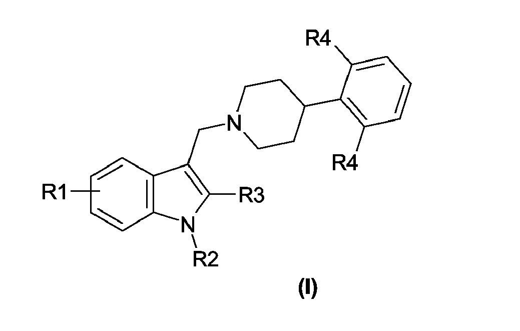 wherein:
**R1** is hydrogen; halogen; C₁₋₆alkyl; perhaloC₁₋₆alkyl; aryl;
**R2** and **R3** are independently hydrogen; (CH₂)ₙOH; (CH₂)ₙCONRₐR_{b}; (CH₂)ₙNHCORₐ; (CH₂)ₙCONHSO₂Rₐ; (CH₂)ₙNHSO₂Rₐ; aryl; arylC₁₋₆alkyl,
   where:
n = 0-4;
Rₐ and R_{b} are independently hydrogen; linear or branched C₁₋₆alkyl optionally substituted one or more times with hydroxy or C₁₋₆alkoxy; C₃₋₆cycloalkyl, aryl; arylC₁₋₆alkyl; or Rₐ and R_{b}, together with the nitrogen atom to which they are attached, may form a heterocycle optionally substituted with =O or C₁₋₆ alkyl containing from 1 to 3 heteroatoms selected among O, S, N, N-Rₐ , where Rₐ is as above defined;
with the proviso that R2 e R3 are not simultaneously hydrogen, and when R2 or R3 is aryl, R3 or R2 is not hydrogen;
**R4** is methyl or chloro.

The term "aryl" as used herein includes the C₅₋₁₀aryl groups, in particular phenyl and naphthyl; wherever present, said aryl may be substituted one or more times by halogen, C₁₋₆alkoxy, C₁₋₆alkyl, hydroxy, amino, C₁₋₆alkylamino, di(C₁₋₆alkyl)amino, aminoC₁₋₆alkyl, (C₁₋₆alkyl)aminoC₁₋₆alkyl, di(C₁₋₆alkyl)aminoC₁₋₆alkyl, aryl or perhaloC₁₋₆alkyl.

The C₁₋₆alkyl groups can be linear or branched and are preferably C₁₋₂alkyl groups, more preferably methyl.

The term "halogen" includes the iodine, chlorine, bromine and fluorine groups, especially chlorine, fluorine and bromine.

Preferably, R1 is hydrogen or halogen; more preferably, R1 is hydrogen or fluoro.

Preferably, R2 is hydrogen, (CH₂)ₙOH where n = 2-4, or (CH2)ₙCONRₐR_{b} where n = 1-4; more preferably, R2 is hydrogen, 2-hydroxyethyl, aminocarbonylmethyl;

Preferably, R3 is aryl, (CH₂)ₙCONRₐR_{b},(CH₂)ₙNHCORₐ, (CH₂)ₙCONHSO₂R_{a,} or (CH₂)ₙNHSO₂Rₐ; more preferably, R3 is phenyl, CONRₐR_{b}.

Whenever present as a part of R3, the sub-group -NRₐR_{b} preferably represents one of the following moieties:

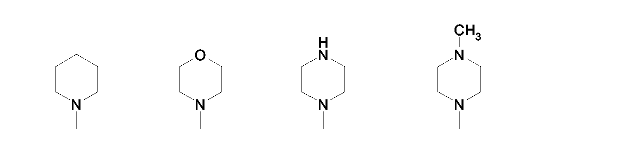

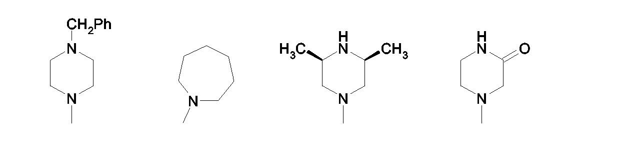

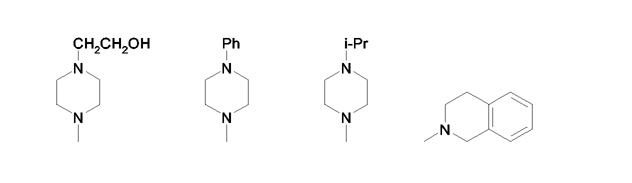

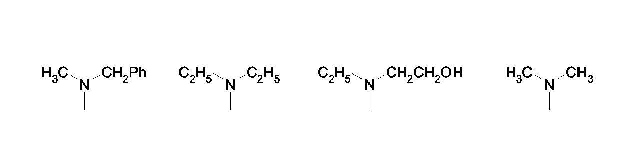

Specific compounds of formula (I) according to the present invention (of which each also comprises the corresponding salts such as hydrochloride or trifluoroacetate), and hydrates, are the following:
3-[4-(2,6-Dimethyl-phenyl)-piperidin-1-ylmethyl]-1H-indole-2-carboxylic acid methylamide;
3-[4-(2,6-Dimethyl-phenyl)-piperidin-1-ylmethyl]-1H-indole-2-carboxylic acid benzylamide;
3-[4-(2,6-Dimethyl-phenyl)-piperidin-1-ylmethyl]-1H-indole-2-carboxylic acid phenylamide;
{3-[4-(2,6-Dimethyl-phenyl)-piperidin-1-ylmethyl]-1H-indol-2-yl}-(4-methyl-piperazin-1-yl)-methanone;
3-[4-(2,6-Dimethyl-phenyl)-piperidin-1-ylmethyl]-1H-indole-2-carboxylic acid amide;
3-[4-(2,6-Dimethyl-phenyl)-piperidin-1-ylmethyl]-1H-indole-2-carboxylic acid dimethylamide trifluoroacetate;
3-[4-(2,6-Dimethyl-phenyl)-piperidin-1-ylmethyl]-1H-indole-2-carboxylic acid cyclohexylamide;
{3-[4-(2,6-Dimethyl-phenyl)-piperidin-1-ylmethyl]-1H-indol-2-yl}-piperidin-1-yl-methanone;
{3-[4-(2,6-Dimethyl-phenyl)-piperidin-1-ylmethyl]-1H-indol-2-yl}-piperazin-1-yl-methanone dihydrochloride;
{3-[4-(2,6-Dimethyl-phenyl)-piperidin-1-ylmethyl]-1H-indol-2-yl}-morpholin-4-yl-methanone;
{3-[4-(2,6-Dimethyl-phenyl)-piperidin-1-ylmethyl]-1H-indol-2-yl}-(cis-3,5-dimethyl-piperazin-1-yl)-methanone;
3-[4-(2,6-Dimethyl-phenyl)-piperidin-1-ylmethyl]-1H-indole-2-carboxylic acid benzyl-methyl-amide;
(3,4-Dihydro-1 H-isoquinolin-2-yl)-{3-[4-(2,6-dimethyl-phenyl)-piperidin-1-ylmethyl]-1H-indol-2-yl}-methanone;
Azepan-1-yl-{3-[4-(2,6-dimethyl-phenyl)-piperidin-1-ylmethyl]-1H-indol-2-yl}-methanone;
3-[4-(2,6-Dimethyl-phenyl)-piperidin-1-ylmethyl]-1H-indole-2-carboxylic acid diethylamide;
(4-Benzyl-piperazin-1-yl)-{3-[4-(2,6-dimethyl-phenyl)-piperidin-1-ylmethyl]-1H-indol-2-yl}-methanone;
4-{3-[4-(2,6-Dimethyl-phenyl)-piperidin-1-ylmethyl]-1H-indole-2-carbonyl}-piperazin-2-one;
{3-[4-(2,6-Dimethyl-phenyl)-piperidin-1-ylmethyl]-1H-indol-2-yl}-(4-phenyl-piperazin-1-yl)-methanone;
{3-[4-(2,6-Dimethyl-phenyl)-piperidin-1-ylmethyl]-1H-indol-2-yl}-(4-isopropyl-piperazin-1-yl)-methanone ditrifluoroacetate;
3-[4-(2,6-Dimethyl-phenyl)-piperidin-1-ylmethyl]-1H-indole-2-carboxylic acid ethyl-(2-hydroxy-ethyl)-amide trifluoroacetate;
{3-[4-(2,6-Dimethyl-phenyl)-piperidin-1-ylmethyl]-1H-indol-2-yl}-[4-(2-hydroxyethyl)-piperazin-1-yl]-methanone;
{3-[4-(2,6-Dichloro-phenyl)-piperidin-1-ylmethyl]-1H-indol-2-yl}-piperidin-1-ylmethanone;
{3-[4-(2,6-Dichloro-phenyl)-piperidin-1-ylmethyl]-1H-indol-2-yl}-morpholin-4-ylmethanone hydrochloride;
(4-Benzyl-piperazin-1-yl)-{3-[4-(2,6-dichloro-phenyl)-piperidin-1-ylmethyl]-1H-indol-2-yl}-methanone dihydrochloride;
4-{3-[4-(2,6-Dichloro-phenyl)-piperidin-1-ylmethyl]-1H-indole-2-carbonyl}-piperazin-2-one.;
2-{3-[4-(2,6-Dichloro-phenyl)-piperidin-1-ylmethyl]-2-phenyl-indol-1-yl}-acetamide.

The compounds of formula (I) can exhibit stereoisomerism because of the presence of chiral atoms and/or multiple bonds. The present invention therefore extends to stereoisomers of the compounds of the formula (I), including racemes, enantiomers, diastereoisomers and geometric isomers.

It has been found that, when a compound of formula (I) exhibits optical isomerism, an enantiomer possesses a greater affinity for the ORL-1 receptor than its antipod.

Consequently, the present invention also provides an enantiomer of a compound of formula (I).

In a further aspect, the present invention provides a mixture of enantiomers of a compound of formula (I) where an enantiomer is present in a proportion greater than its antipod.

The subject invention also includes isotopically-labelled compounds, which are identical to those recited in formula (I) and following, but for the fact that one or more atoms are replaced by an atom having an atomic mass or mass number different from the atomic mass or mass number usually found in nature.

Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I and ¹²⁵I.

Compounds of the present invention and pharmaceutically acceptable salts of said compounds that contain the aforementioned isotopes and/or other isotopes of other atoms are within the scope of the present invention. Isotopically-labelled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I and following of this invention can generally be prepared by carrying out the procedures disclosed in the Schemes and/or in the Examples below, by substituting a readily available isotopically labelled reagent for a non-isotopically labelled reagent.

The present invention also provides processes for preparing the compounds of formula (I).

Compounds of formula (I) in which R2 is hydrogen, hereinafter referred as formula (Ia), can be obtained reacting a compound of formula (II), 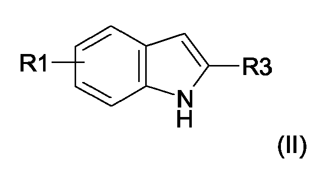 in which R1 and R3 have the meanings described for formula (I), with formaldehyde and a compound of formula (III), 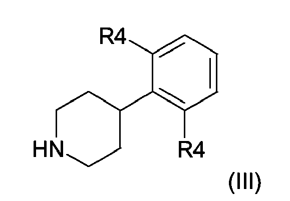 in which R4 has the meanings described for formula (I).

This reaction is typically a Mannich reaction, as described in standard reference texts of synthetic methodology, such as J. *March, Advanced Organic Chemistry, 3rd Edition (1985), Wiley Interscience*.

In particular, compounds of formula (Ia) can be prepared in accordance with scheme 1, starting from compounds of formula (II), formaldehyde and an amine of formula (III).

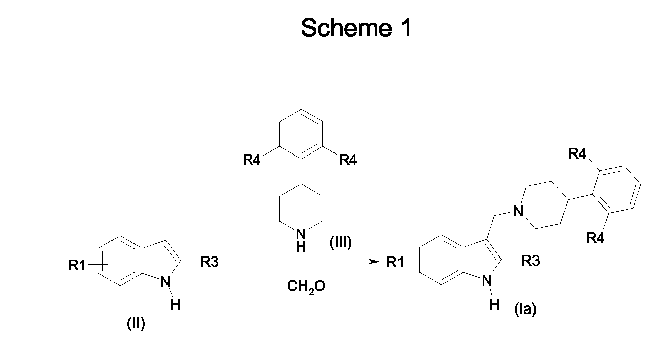

In a typical procedure, an amine of formula (III) is dissolved in a suitable solvent, such as for example methanol or dioxane or a mixture of both, to which solution an aqueous solution of formaldehyde and acetic acid are added. After a suitable time, typically between 5 min and 1 h, there is added to the preceding reaction mixture a solution of an indole of formula (II) in a suitable solvent, such as for example methanol or dioxane or a mixture of both, while maintaining the temperature of the resultant solution generally between 0°C and ambient temperature. The reaction mixture is stirred for a suitable time, typically between 1 h and 96 h, at a suitable temperature, typically between 0°C and 80°C, after which it is processed by known methods.

Two preferred processing procedures are here indicated as procedure A and procedure B.

In procedure A, water is added to the reaction mixture followed by a solution of a suitable base, such as aqueous ammonium hydroxide or sodium hydroxide, until basic pH is reached, after which it is extracted with a suitable organic solvent such as ethyl acetate. The organic phase is collected and dried with, for example, sodium sulfate, and the solvent is removed by evaporation. The crude product can be purified, if necessary, by conventional purification methods such as flash chromatography, trituration, crystallization or preparative HPLC.

In procedure B, the reaction mixture is poured onto an acid resin cartridge and eluted with a suitable solvent, such as for example dichloromethane or methanol, to remove non-basic impurities, and then with a solution of a suitable base in a suitable organic solvent such as, for example, a methanolic ammonia solution, to recover the desired compound of formula (I). The solvent is removed by evaporation and the crude product can be purified, if necessary, by conventional purification methods such as flash chromatography, trituration, crystallization or preparative HPLC.

Compounds of formula (II) are known or commercially available compounds or can be prepared by procedures described in standard reference texts of synthesis methodologies such as J. *March, Advanced Organic Chemistry, 3rd Edition (1985), Wiley Interscience.*

The compounds of formula (III) can be prepared by the procedures described in WO 01 /83454.

Compounds of formula (I) in which R2 is different from hydrogen, hereinafter referred as formula (Ib), can be prepared as follows: a compound of formula (II) is treated in accordance with the two following steps, which can take place in any order:
a) reaction with formaldehyde and a compound of formula (III)
b) reaction with a compound of formula R2-X, in which R2 is as defined in formula (I) and X is a suitable leaving group,
   thus obtaining the compounds of formula (Ib).

If the steps are carried out in the order (a) → (b), the compound of formula (II) is firstly functionalized in position 3 by reaction with formaldehyde and the compound of formula (III); the 3-functionalized intermediate obtained is then N-alkylated in position 1 of the indole ring by treatment with the compound R2-X, to obtain the final compound of formula (Ib).

If the steps are carried out in the reverse order (b) → (a), the compound of formula (II) is firstly N-alkylated in position 1 of the indole ring by reaction with the compound R2-X; the N-alkylated intermediate obtained is then 3-functionalized by reaction with formaldehyde and the compound of formula (III), to obtain the final compound of formula (Ib).

Step (a) is effected preferably by the Mannich reaction, in the previously detailed manner.

Step (b) is a nucleophilic reaction which can be effected by commonly known methods; in particular it is effected by reacting the compound of formula (II) (or, as illustrated in the following Scheme 2, its 3-substituted derivative resulting from step (a)) with a strong base and then treating the resultant indolyl anion with said compound of formula R2-X.

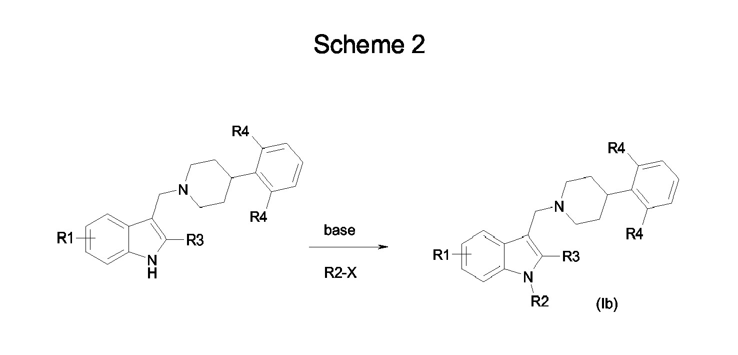

In a typical procedure, a suitable base such as sodium hydride, is added under an inert atmosphere, typically of argon or nitrogen, to a solution of a compound of formula (II) or its 3-substituted derivative in a suitable anhydrous solvent, such as dimethylformamide, at a suitable temperature, typically between 0°C and ambient temperature. After a suitable time, typically between 15 min and 1 h, a suitable alkylating agent of formula R2-X is added to the reaction mixture, either as such or dissolved in a suitable anhydrous solvent such as dimethylformamide; if necessary, further additions of alkylating agent can be made. The resultant reaction mixture is stirred at a suitable temperature, typically ambient temperature, for a suitable time, typically between 1 h and 20 h. The procedure can be carried out by known methods. Two preferred working procedures are here indicated as procedure A and procedure B.

In procedure A, water is added to the reaction mixture, which is then extracted with a suitable organic solvent such as diethylether. The organic phase is collected and dried with, for example, sodium sulfate, and the solvent is removed by evaporation. The crude product can be purified, if necessary, by conventional purification methods such as flash chromatography, trituration, crystallization and preparative HPLC.

In working procedure B, water is added to the reaction mixture, which is then filtered through a suitable water retention filter, eluting with a suitable solvent such as ethyl acetate. The resultant solution can be concentrated, if necessary, and then poured onto an acid resin cartridge and eluted with a suitable solvent, such as methanol, to remove non-basic impurities, and then with a solution of a suitable base in a suitable organic solvent such as a methanolic solution of ammonia, to recover the desired compound of formula (I). The solvent is removed by evaporation and the crude product can be purified, if necessary, by conventional purification methods such as flash chromatography, trituration, crystallization and preparative HPLC.

Compounds of formula (I) in which R3 is a group (CH₂)ₙCONRₐR_{b} and n = 0, hereinafter referred as formula (Ic), can be prepared in accordance with scheme 3, activating an amine of formula HNRₐR_{b}, where Rₐ and R_{b} have the meanings described for formula (I), with trimethylaluminium and then reacting said activated amine with a compound of formula (IV), 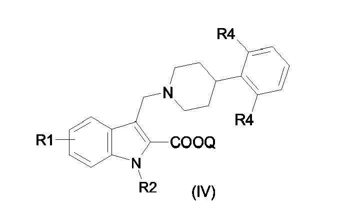 where R1, R2 and R4 have the meanings given for formula (I), and Q is a linear or branched C₁₋₄alkyl, under conditions reported for example in Basha *et al., Tetrahedron Lett*., **18**, 4171, (1977).

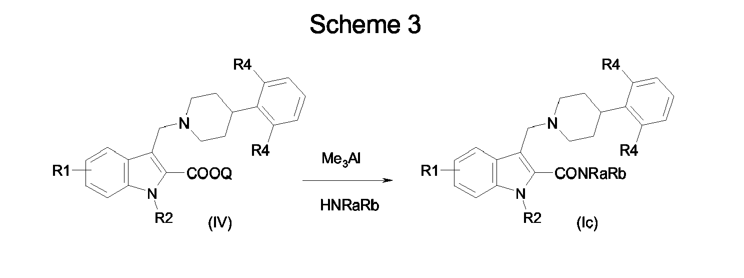

In a typical procedure, a solution of trimethylaluminium in a suitable solvent, such as for example hexane or toluene, is added at a suitable temperature, typically between 0°C and room temperature, to a solution of an amine of formula HNRₐR_{b} dissolved in a suitable anhydrous solvent, such as for example dichloromethane or toluene, under an inert atmosphere, typically of nitrogen or argon. After a suitable time, typically between 5 min and 1 h, there is added to the preceding reaction mixture a solution of a compound of formula (IV) dissolved in a suitable anhydrous solvent, such as for example dichloromethane or toluene, while maintaining the temperature of the resultant solution generally between 0°C and room temperature. The reaction mixture is stirred for a suitable time, typically between 30 min to 48 h, at a suitable temperature, typically between room temperature and 110°C, after which water is added and the reaction mixture is extracted with a suitable solvent such as ethyl acetate or dichloromethane. The organic phase is collected and dried with, for example, sodium sulfate and the solvent is removed by evaporation. The crude product can be purified, if necessary, by conventional purification methods such as flash chromatography, trituration, crystallization and preparative HPLC.

Amines of formula HNRₐR_{b} are known or commercially available compounds or can be prepared by procedures described in standard reference texts of synthesis methodologies such as J. *March, Advanced Organic Chemistry, 3rd Edition (1985), Wiley Interscience.*

Compounds of formula (IV) can be prepared as described in scheme 4, starting from compounds of formula (V), an amine of formula (III) and formaldehyde, under the experimental conditions detailed above for the synthesis of compounds of formula (Ia).

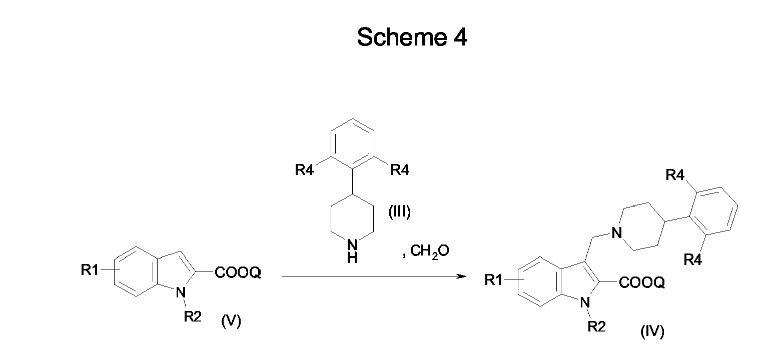

Compounds of formula (V) are known or commercially available compounds or can be prepared by procedures described in standard reference texts of synthesis methodologies such as J. *March, Advanced Organic Chemistry, 3rd Edition (1985), Wiley Interscience.*

Compounds of formula (I) in which R2 is (CH₂)ₙCONRₐR_{b} and n = 1-4, hereinafter referred as formula (Id), can be prepared in accordance with scheme 5, reacting a compound of formula (Ia), with a compound of formula X-(CH₂)ₙCOOQ, where X is a suitable leaving group as described above, n = 1-4 and Q is as defined above, under alkylating conditions described above, to give compounds of formula (VI), hydrolysing the ester group to the corresponding carboxylic acid of formula (VII) and then reacting a suitably activated form of acid (VII) with an amine of formula HNRₐR_{b}, where Rₐ and R_{b} have the meanings described for formula (I).

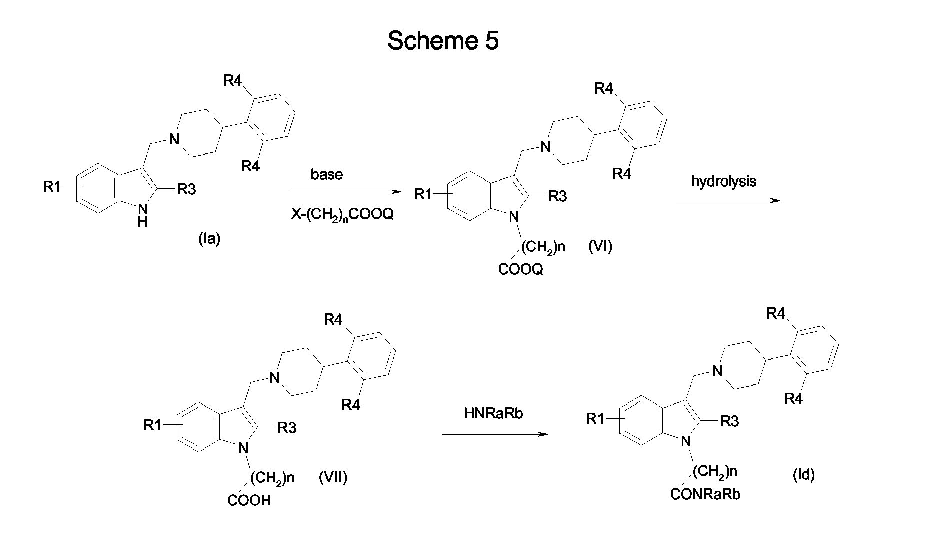

Hydrolysis of compounds of formula (VI) can take place under basic (for example, sodium or potassium hydroxide solution) or acidic (for example, trifluoroacetic acid) conditions.

Activation of the compound of formula (VII), effected before reacting with the compounds of formula HNRₐR_{b}, can suitably take place by forming the corresponding acyl halides, for example by reaction with oxalyl chloride or thionyl chloride; alternatively, the compounds of formula (VII) can also be activated using activating agents such as 1,1'-carbonyldiimidazole, dicyclohexylcarbodiimide/1-hydroxybenzotriazole, N-ethyl-N'-(3-dimethylamino-propyl)carbodiimide/1-hydroxybenzotriazole, or O-benzotriazol-1-yl-N,N,N',N'-tetramethyluronium hexafluorophosphate.

In a typical procedure, trifluoroacetic acid is added to a solution of a compound of formula (VI) in a suitable solvent, such as for example dichloromethane, at a suitable temperature, typically between 0°C and room temperature and the reaction mixture is stirred for a suitable time, typically between 1 and 24 h, after which the reaction mixture is evaporated to dryness. The crude compound of formula (VII) is dissolved in a suitable solvent, such as for example acetonitrile, and an activating agent such as 1,1'-carbonyldiimidazole is added to this solution and the reaction mixture is stirred for a suitable time, typically between 1 and 24 h, at a suitable temperature, typically between room temperature and 80°C, then an amine of general formula HNRaRb is added to the solution. The reaction mixture is stirred for a suitable time, generally between 15 min and 8 h, and at a suitable temperature, typically between room temperature and 80°C, then water is added and the reaction mixture is extracted with a suitable solvent, such as ethyl acetate or dichloromethane. The organic phase is collected and dried with, for example, sodium sulfate and the solvent is removed by evaporation. The crude product can be purified, if necessary, by conventional purification methods such as flash chromatography, trituration, crystallization and preparative HPLC.

Alternatively, compounds of general formula (Id) can be prepared in accordance with scheme 6, activating an amine of formula HNRₐR_{b}, where Rₐ and R_{b} have the meanings described for formula (I), with trimethylaluminium, and then reacting said activated amine with a compound of formula (VI), under conditions reported above for the synthesis of compounds of general formula (Ic).

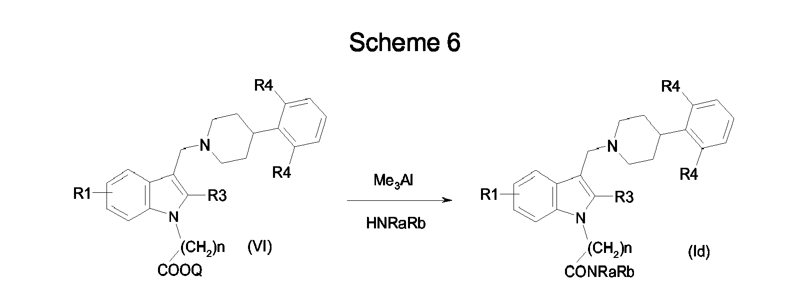

As aforestated, the compounds of formula (I) are ligands for the ORL-1 receptor. Hence, a compound of formula (I) is provided as active therapeutic substance.

According to another aspect of the present invention a method is provided for modulating the activity of the ORL-1 receptor in a human or animal patient in need thereof, comprising administering to the human or animal patient an effective quantity of a compound of formula (I).

Another aspect of the present invention provides the use of a compound of formula (I) in preparing a medicament for human or animal administration, useful for modulating the activity of the ORL-1 receptor.

Said compounds of formula (I) can be agonists or antagonists of the ORL-1 receptor.

The compounds of the invention are therefore useful in the therapy and/or prophylaxis of all those illnesses dependent on modulation of the ORL-1 receptor. Accordingly they can be used as analgesics in man or animals in treating or preventing, for example, acute pain, chronic neuropathic or inflammatory pain, including post-herpes neuralgia, neuralgia, diabetic neuropathy and post-infarct pain; visceral pain including that associated with irritable bowel syndrome, dysmenorrhea, and hyperreflexia of the bladder; osteoarthritis, back pain, labour pain in childbirth.

Said compounds can further be useful in the treatment or prophylaxis of gastrointestinal disorders including irritable bowel syndrome, and symptoms associated with non-ulcerous dyspepsia and gastro-oesophageal reflux; diseases of the immune system; dysfunctions of the cardiovascular system such as infarct, congestive cardiac insufficiency and pathologies associated with alterations of arterial pressure; diseases of the excretory system, such as altered diuresis, water homeostasis and sodium excretion, syndrome of inappropriate anti-diuretic hormone secretion (SIADH); sexual dysfunctions including impotence and frigidity; cirrhosis with ascites.

These compounds can also be useful in the treatment or prophylaxis of disorders of the respiratory tract such as cough, asthma, adult respiratory distress syndrome (ARDS), altered pulmonary function, including chronic obstructive pulmonary disease.

Compounds of the invention are further useful in the treatment of central nervous system disorders where ORL-1 receptors are involved. In particular in the treatment or prevention of major depressive disorders including bipolar depression, unipolar depression, single or recurrent major depressive episodes with or without psychotic or catatonic features, catatonic features, melancholic features, atypical features or postpartum onset, the treatment of anxiety and the treatment of panic disorders. The term anxiety includes anxiety disorders, such as panic disorders with or without agoraphobia, agoraphobia, phobias, for example, social phobias or agoraphobia, obsessive-compulsive disorder, stress disorders including post-traumatic stress disorders, generalised anxiety disorders, acute stress disorders and mixed anxiety-depression disorders. Other mood disorders encompassed within the term major depressive disorders include dysthymic disorder with early or late onset and with or without atypical features, neurotic depression, post traumatic stress disorders, post operative stress and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.

Compounds of the invention are also useful in the treatment or prevention of dementia as such, e.g. vascular dementia and dementia associated with AIDS; they are further effective in treating or preventing motor damage and neurodegeneration due to Alzheimer's disease; senile dementia, Parkinson's disease, disorders due to defective motor coordination or other neurodegenerative pathologies.

Compounds of the invention are also useful in the treatment or prevention of epilepsy; schizophrenic disorders including paranoid schizophrenia, disorganised schizophrenia, catatonic schizophrenia, undifferentiated schizophrenia, residual schizoprenia.

Compounds of the invention are also useful for the treatment of dysfunction of appetite and food intake and in circumstances such as anorexia, anorexia nervosa bulimia, and metabolic disorders such as obesity.

Compounds of the invention are also useful in the treatment of sleep disorders including dysomnia, insomnia, sleep apnea, narcolepsy, and circadian rhythmic disorders.

Compounds of the invention are also useful in the treatment or prevention of cognitive disorders. Cognitive disorders include dementia, amnestic disorders, memory loss, and cognitive disorders not otherwise specified. Furthermore compounds of the invention are also useful as memory and/or cognition enhancers in healthy humans with no cognitive and/or memory deficit.

Compounds of the invention are also useful in the treatment of drug abuse, tolerance to and dependence on a number of substances. For example, they are useful in the treatment of dependence on nicotine, alcohol, caffeine, phencyclidine (phencyclidine like compounds), or in the treatment of tolerance to and dependence on opiates (e.g. cannabis, heroin, morphine) or benzodiazepines; in the treatment of cocaine, sedative ipnotic, amphetamine or amphetamine- related drugs (e.g. dextroamphetamine, methylamphetamine) addiction or a combination thereof.

The compounds of formula (I) can be prepared in the form of salts or hydrates. Suitable salts are pharmaceutically acceptable salts. Suitable hydrates are pharmaceutically acceptable hydrates.

An effective quantity of compound of the invention depends on factors such as the nature or seriousness of the illness or illnesses to be treated and on the weight of the patient. In all cases a unit dose normally contains from 0.1 to 50 mg, for example from 0.5 to 10 mg, of the compound. Unit doses are normally administered one or more times per day, for example, 2, 3 or 4 times a day, in particular from 1 to 3 times per day, so that the total daily dose is normally, for an adult of 70 kg, between 0.1 and 50 mg, for example between 0.1 and 5 mg, i.e. in the approximate range of 0.001 to 1 mg/kg/day, in particular between 0.005 and 0.2 mg/kg/day. For oral or parenteral administration, it is highly preferred that the compound be administered in the form of unit dose composition for example, in the form of unit dose oral or parenteral composition.

These compositions are prepared by mixing and are suitably adapted to oral or parenteral administration, and as such can be in the form of tablets, capsules, oral preparations, powders, granules, lozenges, reconstitutable powders, injectable or infusible liquid solutions, suspensions or suppositories.

Tablets and capsules for oral administration are normally presented in unit dose form, and contain conventional excipients such as binders, fillers, diluents, compressing agents, lubricants, detergents, disintegrants, colorants, aromas and wetting agents. The tablets can be covered by methods well known in the art.

Suitable fillers include cellulose, mannitol, lactose and other similar agents. Suitable disintegrants include starch, polyvinylpyrrolidone and starch derivatives such as sodium glycolate starch. Suitable lubricants include, for example, magnesium stearate. Suitable wetting agents include sodium laurylsulfate.

These solid oral compositions can be prepared by conventional methods of mixing, filling or compression. The mixing operations can be repeated to disperse the active component in compositions containing large quantities of fillers. These operations are conventional.

Oral liquid preparations can be in the form, for example, of aqueous or oily suspensions, solutions, emulsions, syrups, or elixirs, or can be presented as a dry product for reconstitution with water or with a suitable carrier before use. These liquid preparations can contain conventional additives such as suspending agents, for example sorbitol, syrup, methylcellulose, gelatin, hydroxyethylcellulose, carboxymethylcellulose, aluminium stearate gel or hydrogenated edible fats, emulsifying agents, for example lecithin, sorbitan monooleate, or acacia; non-aqueous carriers (which can include edible oils), for example almond oil, fractionated coconut oil, oily esters such as glycerine esters, propylene glycol, or ethyl alcohol; preservatives, for example methyl or propyl p-hydroxybenzoate or sorbic acid, and if desired, conventional aromas or colorants.

Oral formulations also include conventional slow-release formulations, such as tablets or granules having an enteric coating.

For parenteral administration, fluid dose units can be prepared, containing the compound and a sterile carrier. The compound, depending on the carrier and the concentration, can be suspended or dissolved. Parenteral solutions are normally prepared by dissolving the compound in a carrier and sterilizing by means of a filter, before filling suitable vials or ampoules and sealing. Advantageously, adjuvants such as local anaesthetics, preservatives and buffer agents can also be dissolved in the carrier. To increase stability, the composition can be frozen after filling the vial and removing the water under vacuum. Parenteral suspensions are prepared substantially in the same manner, with the difference that the compound can be suspended in the carrier instead of dissolved, and be sterilized by exposure to ethylene oxide before suspension in the sterile carrier. Advantageously, a surfactant or a wetting agent can be included in the composition to facilitate uniform distribution of the compound of the invention.

As in common practise, the compositions are normally accompanied by written or printed instructions, for use in the treatment in question.

Consequently, another aspect of the present invention also provides a pharmaceutical composition comprising a compound of formula (I), or a pharmaceutically suitable salt or hydrate thereof, and a pharmaceutically acceptable carrier.

The invention will now be illustrated by means of the following non-limiting examples.

### EXPERIMENTAL PART

### Preparation 1

### 3-[4-(2,6-Dichloro-phenyl)-piperidin-1-ylmethyl]-1H-indole-2-carboxylic acid ethyl ester

471 mg (2.05 mmol) of 4-(2,6-dichloro-phenyl)-piperidine were dissolved in 8 mL of MeOH:dioxane mixture (8:2 respectively); 0.169 mL (2.25 mmol) of CH₂O (37% aqueous solution) and 0.141 mL (2.45 mmol) of glacial AcOH were added at room temperature. After stirring for 30 minutes, a solution of 503 mg (2.66 mmol) of 1 H-indole-2-carboxylic acid ethyl ester in 20 mL of MeOH:dioxane mixture (8:2 respectively) was added. The reaction mixture was heated to 50°C for 5 h, then 2 mL of AcOH were added and heating was continued for 13 h. The volatiles were removed *in vacuo,* the residue was taken up in H₂O/AcOEt and concd. NH₄OH was added up to basic pH. After extraction with AcOEt the organic phase was collected, dried with Na₂SO₄ and the solvent removed *in vacuo.* The crude product was purified by chromatography, eluting with a mixture CH₂Cl₂/MeOH/concd. NH₄OH 100:1:0.1 respectively, yielding 410 mg of the title compound.
NMR (300 MHz, CDCl₃, δ ppm): 8.79 (s br, 1 H); 8.04 (d, 1 H); 7.41-7.29 (m, 2H); 7.29-7.11 (m, 3H); 7.01 (dd, 1 H); 4.43 (q, 2H); 4.18 (s br, 2H); 3.48 (tt, 1 H); 3.11 (m, 2H); 2.67 (m, 2H); 2.21 (m, 2H); 1.50 (m, 2H); 1.44 (t, 3H). MS (m/z): 431 (MH⁺).

### Preparation 2

### {3-[4-(2,6-Dichloro-phenyl)-piperidin-1-ylmethyl]-2-phenyl-indol-1-yl}-acetic acid tert-butyl ester

Under a nitrogen atmosphere, 16.5 mg (0.413 mmol) of NaH (60% dispersion in mineral oil) were suspended in 1 mL of dry DMF. After cooling to 0°C, a solution of 150 mg (0.344 mmol) of 3-[4-(2,6-dichloro-phenyl)-piperidin-1-ylmethyl]-2-phenyl-1 H-indole in 1 mL of dry DMF was added dropwise. The reaction mixture was stirred 30 min at the same temperature, then 0.056 mL (0.379 mmol) of *tert-*butyl bromoacetate were added dropwise. The reaction mixture was allowed to warm to room temperature and stirred 16 h. After cooling to 0°C water was added, followed by concd. NH₄OH and the resulting mixture was extracted with Et₂O. The organic layer was dried with Na₂SO₄ and the solvent was removed *in vacuo.* The resulting crude product was purified by chromatography, eluting with CH₂Cl₂ and then with a mixture CH₂Cl₂/AcOEt 7:3 respectively, yielding 134 mg of the title compound.
NMR (300 MHz, CDCl₃, δ ppm): 7.96 (d, 1 H); 7.53-7.40 (m, 5H); 7.31-7.15 (m, 5H); 7.01 (dd, 1 H); 4.55 (s, 2H); 3.64 (s, 2H); 3.45 (tt, 1 H); 3.04 (m, 2H); 2.59 (m, 2H); 2.05 (m, 2H); 1.45 (m, 2H); 1.38 (s, 9H). MS (m/z): 549.1 (MH⁺); 320.2, 264.2, 218.2, 205.1.

Compounds of formula (1) and described in Table 1 were obtained following either general procedure A or general procedure B.

### General procedure A

To a solution of 156 mg (0.82 mmol) of 4-(2,6-dimethyl-phenyl)-piperidine in 5 mL of MeOH:dioxane mixture (8:2 respectively), 0.097 mL (1.29 mmol) of CH₂O (37% aqueous solution) and 2 mL of glacial AcOH were added at room temperature.

After stirring for 30 minutes, a solution of 0.86 mmol of the appropriate 1 H-indole-2-carboxylic acid amide in 10 mL of MeOH:dioxane mixture (8:2 respectively) was added, and the resulting mixture was heated to 80°C for 10 h. Most of the solvent was removed *in vacuo,* then the remaining solution was poured onto crushed ice and the mixture was made basic with 30% NaOH solution and extracted with

AcOEt. The organic phase was dried and the solvent evaporated.

The crude products were purified by chromatography or by preparative HPLC, yielding the compounds described in Examples 1-22 in Table 1.

### General procedure B

Under a nitrogen atmosphere, 0.37 mmol of amine were dissolved in 0.5 mL of dry toluene, then 0.185 mL (0.37 mmol) of a 2M solution of AlMe₃ in toluene were added dropwise at room temperature. After stirring for 45 min, a solution of 80 mg (0.185 mmol) of 3-[4-(2,6-dichloro-phenyl)-piperidin-1-ylmethyl]-1H-indole-2-carboxylic acid ethyl ester (compound described in Preparation 1) in 1 mL of dry toluene was added dropwise. The reaction mixture was heated to reflux for 90 min, then, after cooling to room temperature, water and AcOEt were added. The organic phase was collected, washed with brine, dried and the solvent was removed *in vacuo.*

The crude products were purified by chromatography, yielding the compounds described in Examples 23-25 in Table 1.

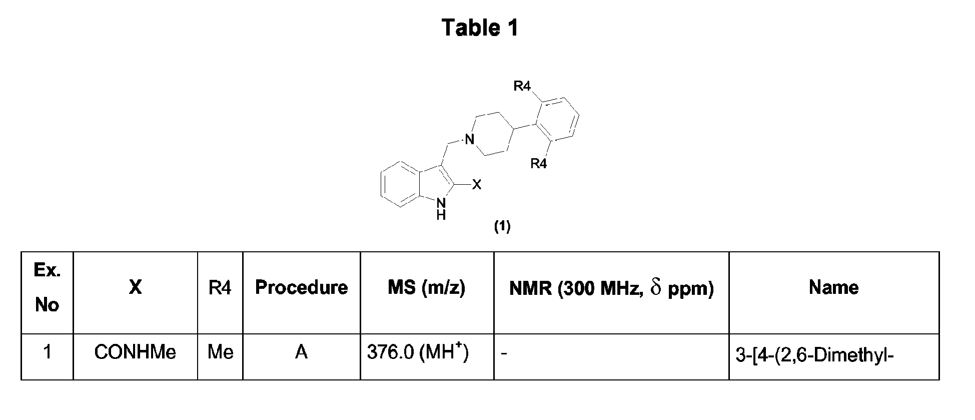

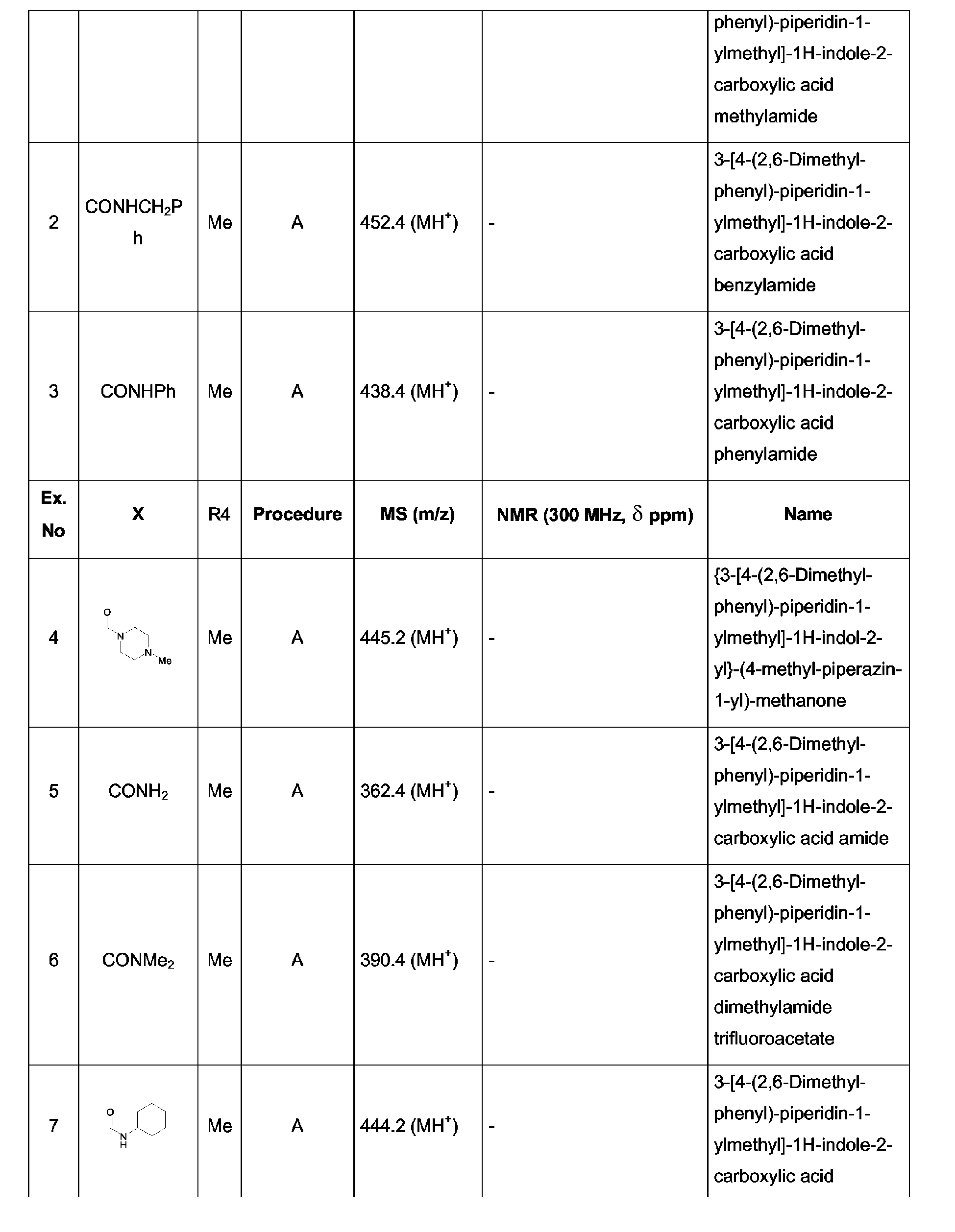

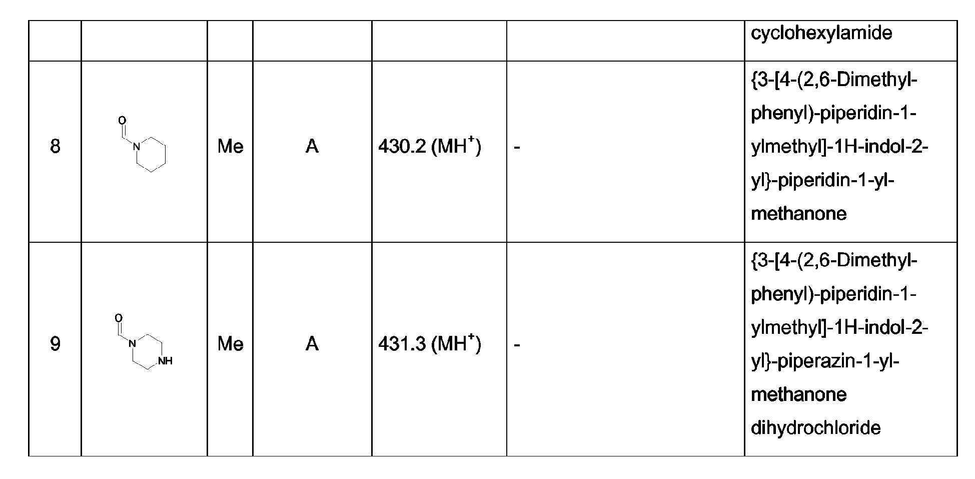

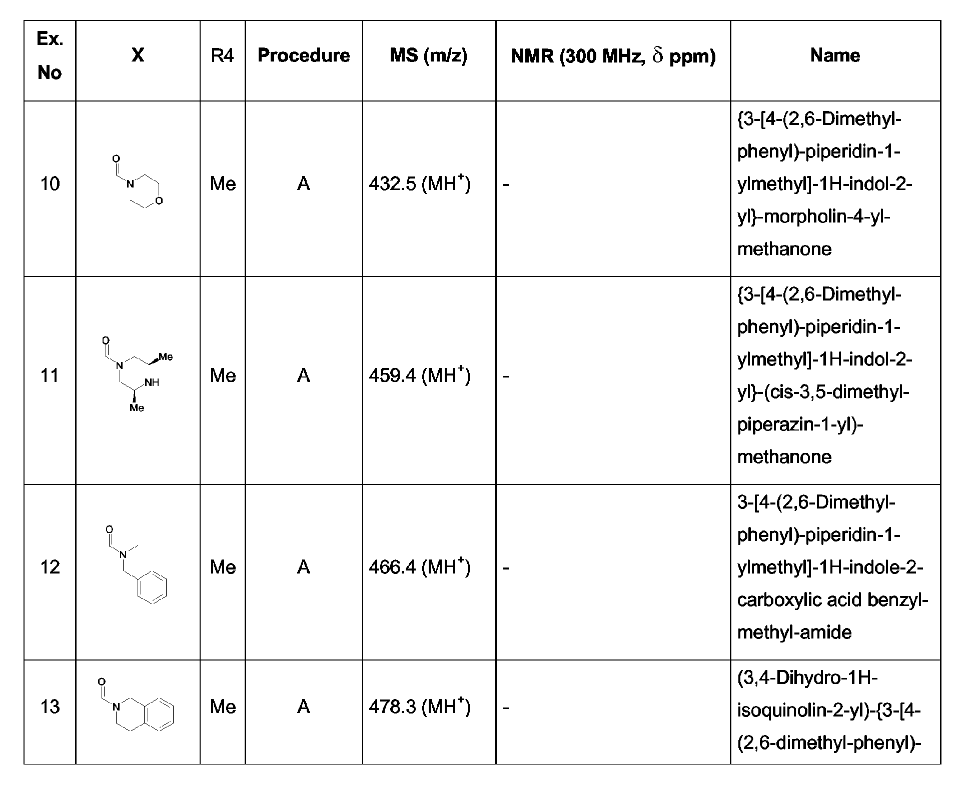

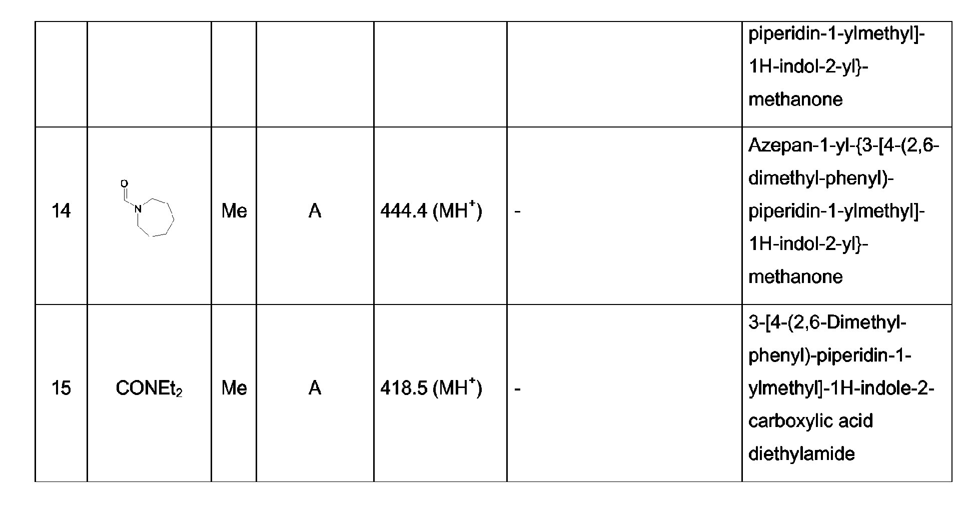

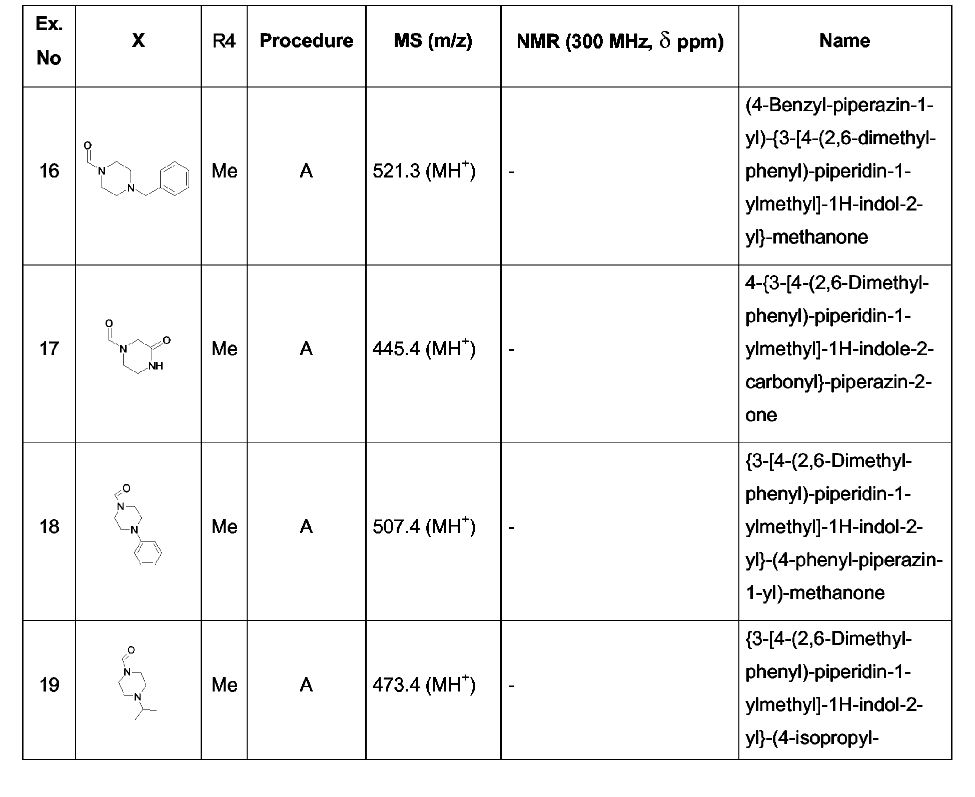

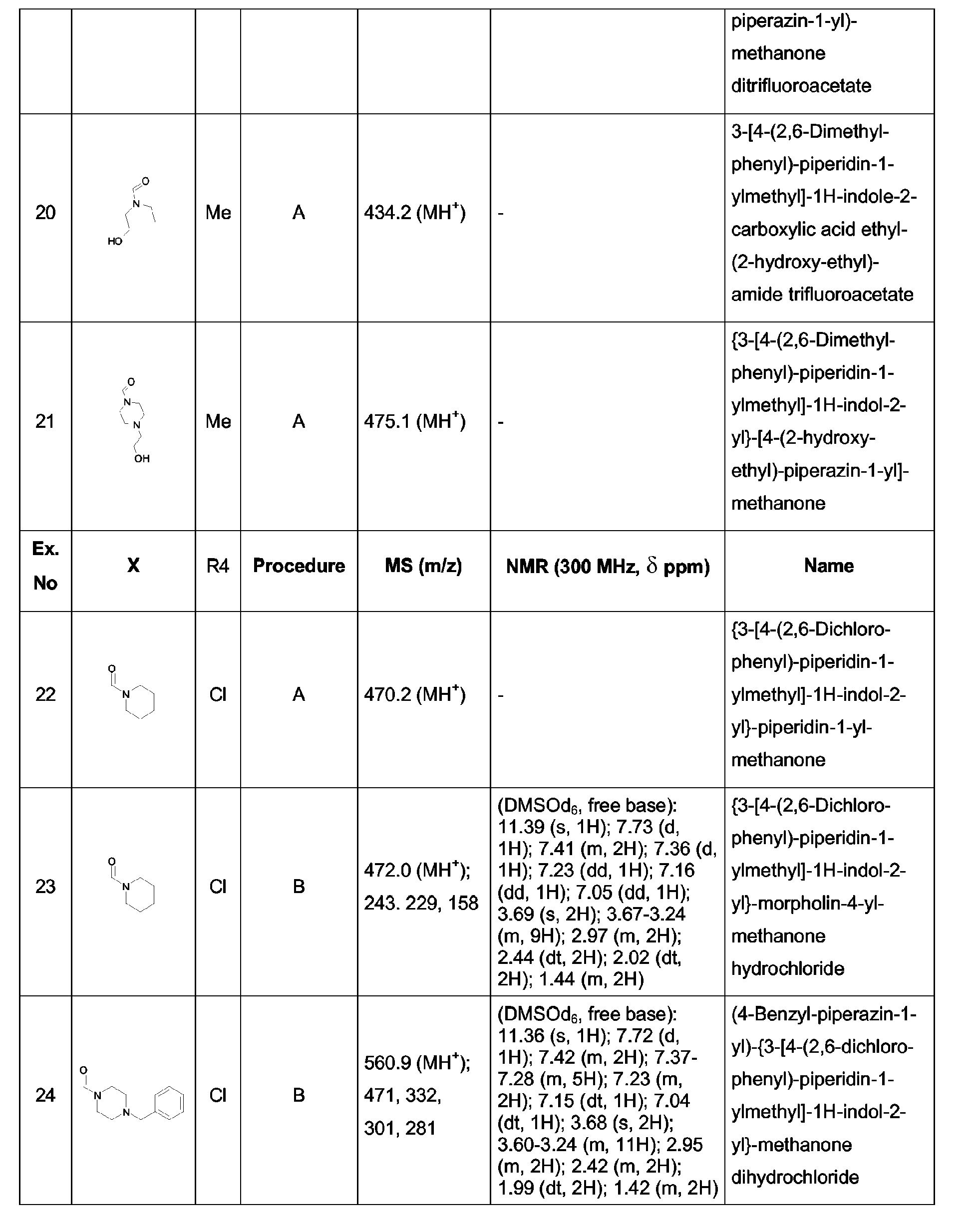

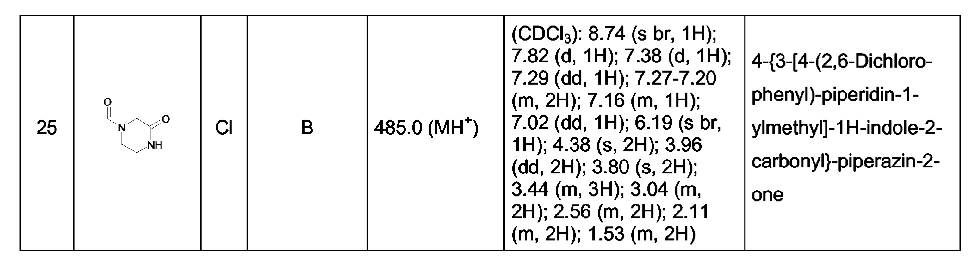

### Example 26

### 2-{3-[4-(2,6-Dichloro-phenyl)-piperidin-1-ylmethyl]-2-phenyl-indol-1-yl}-acetamide

1.5 mL of trifluoroacetic acid were added dropwise to a solution of 134 mg (0.244 mmol) of {3-[4-(2,6-dichloro-phenyl)-piperidin-1-ylmethyl]-2-phenyl-indol-1-yl}-acetic acid tert-butyl ester (compound described in Preparation 2) in 5 mL of CH₂Cl₂ and the reaction mixture was stirred 16 h at room temperature. All the volatiles were removed *in vacuo,* the resulting residue was dissolved in 5 mL of acetonitrile and 79 mg (0.488 mmol) of 1,1'-carbonyldiimidazole were added. After stirring 4 h at room temperature, 2 mL of concd. NH₄OH were added. The reaction mixture was stirred 15 min, then CH₂Cl₂ and water were added. The organic phase was collected, dried and the solvent was removed *in vacuo.* The resulting crude product was purified by chromatography, eluting with a mixture CH₂Cl₂/MeOH/concd. NH₄OH 100:1:0.2 respectively, yielding 94 mg of a solid which was triturated with Et₂O, filtered and dried, yielding 58 mg of the title compound.
NMR (300 MHz, CDCl₃, δ ppm): 7.96 (d, 1 H); 7.53-7.41 (m, 5H); 7.32 (m, 2H); 7.29-7.17 (m, 3H); 7.01 (dd, 1 H); 5.46 (s br, 1 H); 5.34 (s br, 1 H); 4.64 (s, 2H); 3.64 (s, 2H); 3.45 (tt, 1 H); 3.02 (m, 2H); 2.59 (m, 2H); 2.05 (m, 2H); 1.47 (m, 2H).

MS (m/z): 492.0 (MH⁺); 263.1, 218.1, 205.1.

### Pharmacological tests

### Receptor binding studies

The receptor binding studies were carried out on 96-well plates; the incubation medium was Tris HCl pH 7.4 (4°C) containing MgCl₂ (5 mM), EGTA (0.2 mM), BSA (0.1%), with a final volume of 1.0 ml, using as radioligand [³H]-AcRYYRWK-NH₂ (Amersham, 103 Ci/mmol). The samples were incubated at 37°C for 120 min. and were then filtered off via Whatman GF/B filters pre-treated with 0.2% polyethylenimine. The filters were washed three times with Tris HCl buffer pH 7.4 (4°C). The radioactivity present on the filters was measured using a Packard Top Count microplate scintillation counter.

The compounds of formula (I) of the present invention have a binding affinity (Ki) for the ORL-1 receptor in the range from 1 to 1000 nM.

The most potent compounds of formula (I) of the present invention have a binding affinity (Ki) to the ORL-1 receptor in the range from 1 to 100 nM.

### Preparation of membrane for the GTPγS binding test

The entire process was carried out at 4°C. The buffer used consisted of Tris HCl 10 mM, EDTA 0.1 mM, pH 7.4 (4°C) (T.E.).

The cells removed from the culture flask are centrifuged at low speed to remove the growth medium.
1. Resuspend the pellets (a 175 cm² T flask in 0.5-1 ml T.E.).
2. Homogenize the cells using an Ultra-Turrax
3. Centrifuge the homogenate at 1,500 rpm for 10 min at 4°C.
4. Discard the pellets P1
5. Centrifuge the supernatant at 14,000 rpm for 30 min.
6. Discard the supernatant.
7. Resuspend the pellets P2 by suction (microsomal fraction) in 200 ml of T.E. and preserve frozen at -80°C.

To estimate the proteins, dilute the preparation 3 x in T.E. and assay against standard BSA curve 0-2 mg/ml in T.E.. The protein concentration is normally between 1 and 4 mg/ml. The typical yield is 1 mg of proteins per 175 cm² T flask at confluence.

### Binding tests [³⁵S]-GTPγS

The tests were carried out in a 96-well plate using the method modified by Wieland and Jacobs (*Methods Enzymol.,* 1994, **237,** 3-13). The membranes (10 µg per well) and the SPA granules of wheat germ agglutinin (Amersham Pharmacia) (0.5 mg per well) were pre-mixed in buffer solution (HEPES 20 mM, NaCl 100 mM, MgCl₂ 10 mM, pH 7.4, 4°C) and pre-incubated with 10 µM GDP. Increasing concentrations of the compounds to be tested were then incubated with the membrane/granule mixture for 30 min at ambient temperature. 0.3 nM [³⁵S]-GTPγS (1170 Ci/mmol, Amersham) and the ORL-1 agonist were then added.

The total volume of the assay is 100 µl per well. The plates are then incubated at ambient temperature for 30 min under agitation and then centrifuged at 1500 g for 5 min. The quantity of [³⁵S]-GTPγS bound to the membranes was determined by a

Wallac microbeta 1450-Trilux scintillation counter.

The activity of the compound is evaluated as inhibition of [³⁵S]-GTPγS binding stimulation induced by the agonist.

The pIC50 values are determined as the concentration of compound which causes a 50% inhibition of the agonist response.

## Claims

1. Compound of formula (I) 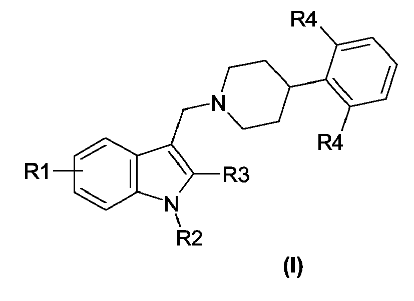 wherein:
**R1** is hydrogen, halogen, C₁₋₆alkyl, perhaloC₁₋₆alkyl, or aryl;
**R2** and **R3** are independently hydrogen, (CH₂)ₙOH, (CH₂)ₙCONRₐR_{b}, (CH₂)ₙNHCORₐ, (CH₂)ₙCONHSO₂Rₐ, (CH₂)ₙNHSO₂Rₐ, aryl, or arylC₁₋₆alkyl,
where:
n = 0-4;
Rₐ and R_{b} are independently hydrogen, linear or branched C₁₋₆alkyl optionally substituted one or more times with hydroxy or C₁₋₆alkoxy, C₃₋₆cycloalkyl, aryl, or arylC₁₋₆alkyl; or Rₐ and R_{b}, together with the nitrogen atom to which they are attached form a heterocycle optionally substituted with =O or C₁₋₆ alkyl containing from 1 to 3 heteroatoms selected among O, S, N, N- Rₐ , where Rₐ is as above defined;
and where each aryl may be substituted one or more times by halogen, C₁₋₆alkoxy, C₁₋₆alkyl, hydroxy, amino, C₁₋₆alkylamino, di(C₁₋₆alkyl)amino, aminoC₁₋₆alkyl, (C₁₋₆alkyl)aminoC₁₋₆alkyl, di(C₁₋₆alkyl)aminoC₁₋₆alkyl, aryl or perhaloC₁₋₆alkyl;
R4 is methyl or chloro.
with the proviso that R2 e R3 are not simultaneously hydrogen, and when R2 or R3 is aryl, R3 or R2 is not hydrogen

2. Compounds according to claim 1, wherein R1 is hydrogen or halogen.

3. Compounds according to claim 1, where R2 is hydrogen, (CH₂)ₙOH where n = 2-4, or (CH₂)ₙCONRₐR_{b} where n = 1-4.

4. Compounds according to claim 1, where R3 is aryl, (CH₂)ₙCONRₐR_{b}, (CH₂)ₙNHCORₐ, (CH₂)ₙCONHSO₂Rₐ, or (CH₂)ₙNHSO₂Rₐ.

5. Compounds according to claim 1, where R3 is phenyl, CONRₐR_{b}.

6. Compounds according to claim 1, where the sub-group -NRₐR_{b}, part of R3, represents one of the following structures:

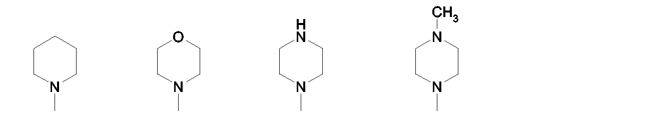

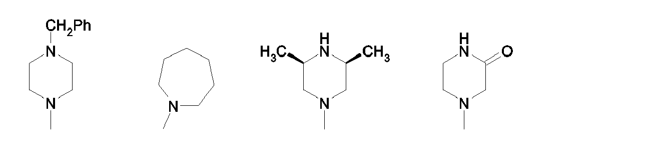

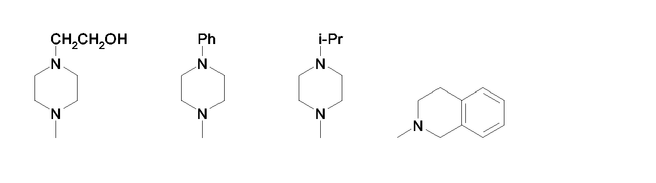

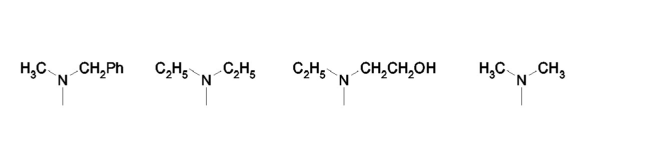

7. Compounds according to claim 1, selected from:
3-[4-(2,6-Dimethyl-phenyl)-piperidin-1-ylmethyl]-1H-indole-2-carboxylic acid methylamide;
3-[4-(2,6-Dimethyl-phenyl)-piperidin-1-ylmethyl]-1H-indole-2-carboxylic acid benzylamide;
3-[4-(2,6-Dimethyl-phenyl)-piperidin-1-ylmethyl]-1H-indole-2-carboxylic acid phenylamide;
{3-[4-(2,6-Dimethyl-phenyl)-piperidin-1-ylmethyl]-1H-indol-2-yl}-(4-methyl-piperazin-1-yl)-methanone;
3-[4-(2,6-Dimethyl-phenyl)-piperidin-1-ylmethyl]-1H-indole-2-carboxylic acid amide;
3-[4-(2,6-Dimethyl-phenyl)-piperidin-1-ylmethyl]-1H-indole-2-carboxylic acid dimethylamide trifluoroacetate;
3-[4-(2,6-Dimethyl-phenyl)-piperidin-1-ylmethyl]-1H-indole-2-carboxylic acid cyclohexylamide;
{3-[4-(2,6-Dimethyl-phenyl)-piperidin-1-ylmethyl]-1H-indol-2-yl}-piperidin-1-ylmethanone;
{3-[4-(2,6-Dimethyl-phenyl)-piperidin-1-ylmethyl]-1H-indol-2-yl}-piperazin-1-ylmethanone dihydrochloride;
{3-[4-(2,6-Dimethyl-phenyl)-piperidin-1-ylmethyl]-1H-indol-2-yl}-morpholin-4-ylmethanone;
{3-[4-(2,6-Dimethyl-phenyl)-piperidin-1-ylmethyl]-1H-indol-2-yl}-(cis-3,5-dimethyl-piperazin-1-yl)-methanone;
3-[4-(2,6-Dimethyl-phenyl)-piperidin-1-ylmethyl]-1H-indole-2-carboxylic acid benzyl-methyl-amide;
(3,4-Dihydro-1 H-isoquinolin-2-yl)-{3-[4-(2,6-dimethyl-phenyl)-piperidin-1-ylmethyl]-1H-indol-2-yl}-methanone;
Azepan-1-yl-{3-[4-(2,6-dimethyl-phenyl)-piperidin-1-ylmethyl]-1H-indol-2-yl}-methanone;
3-[4-(2,6-Dimethyl-phenyl)-piperidin-1-ylmethyl]-1H-indole-2-carboxylic acid diethylamide;
(4-Benzyl-piperazin-1-yl)-{3-[4-(2,6-dimethyl-phenyl)-piperidin-1-ylmethyl]-1H-indol-2-yl}-methanone;
4-{3-[4-(2,6-Dimethyl-phenyl)-piperidin-1-ylmethyl]-1H-indole-2-carbonyl}-piperazin-2-one;
{3-[4-(2,6-Dimethyl-phenyl)-piperidin-1-ylmethyl]-1H-indol-2-yl}-(4-phenyl-piperazin-1-yl)-methanone;
{3-[4-(2,6-Dimethyl-phenyl)-piperidin-1-ylmethyl]-1H-indol-2-yl}-(4-isopropyl-piperazin-1-yl)-methanone ditrifluoroacetate;
3-[4-(2,6-Dimethyl-phenyl)-piperidin-1-ylmethyl]-1H-indole-2-carboxylic acid ethyl-(2-hydroxy-ethyl)-amide trifluoroacetate;
{3-[4-(2,6-Dimethyl-phenyl)-piperidin-1-ylmethyl]-1H-indol-2-yl}-[4-(2-hydroxyethyl)-piperazin-1-yl]-methanone;
{3-[4-(2,6-Dichloro-phenyl)-piperidin-1-ylmethyl]-1H-indo1-2-yl}-piperidin-1-ylmethanone;
{3-[4-(2,6-Dichloro-phenyl)-piperidin-1-ylmethyl]-1H-indol-2-yl}-morpholin-4-ylmethanone hydrochloride;
(4-Benzyl-piperazin-1-yl)-{3-[4-(2,6-dichloro-phenyl)-piperidin-1-ylmethyl]-1H-indol-2-yl}-methanone dihydrochloride;
4-{3-[4-(2,6-Dichloro-phenyl)-piperidin-1-ylmethyl]-1H-indole-2-carbonyl}-piperazin-2-one
2-{3-[4-(2,6-Dichloro-phenyl)-piperidin-1-ylmethyl]-2-phenyl-indol-1-yl}-acetamide.

8. Enantiomer of a compound of formula (I) as described in claims 1-7.

9. Mixture of enantiomers of a compound of formula (I) as described in claims 1-8, where an enantiomer is present in greater proportion than its antipod.

10. Compound of formula (I) as defined in claims 1-9, for use as active therapeutic substance.

11. Pharmaceutical composition comprising a compound of formula (I) as defined in claims 1-9, or a pharmaceutically acceptable salt or hydrate thereof, associated with one or more pharmaceutically acceptable excipients.

12. Process for preparing a compound of formula (I), 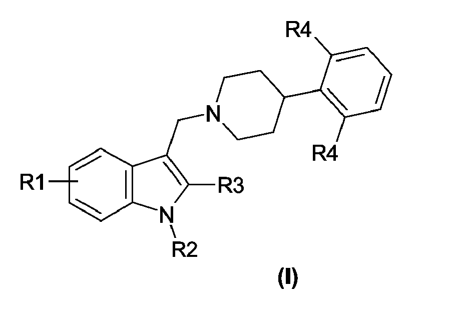 comprising the step of reacting a compound of formula (II) 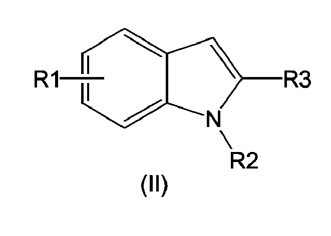 in which R1, R2 and R3 have the meanings described for formula (I), with formaldehyde and a compound of formula (III), 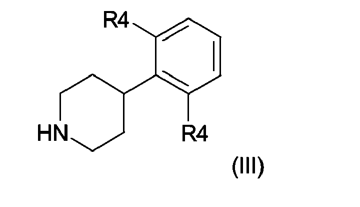 in which R4 has the meanings described for formula (I), thereby obtaining said compound of formula (I).

13. Process according to claim 12 for preparing compounds of formula (I) with R2 different from H, wherein the N-R2 linkage is obtained by N-alkylating the corresponding NH group with a compound of formula R2-X, in which R2 is as above defined and different from hydrogen, and X is a suitable leaving group.

14. Process according to claims 12-13 for preparing compounds of formula (I) wherein R2 and/or R3 is (CH₂)ₙ-CO-NRₐR_{b}, in which such group is formed by reacting the equivalent compound having R2 and/or R3 = COOQ, Q being a linear or branched C1-4 alkyl, with an amine of formula HNRₐR_{b} , in suitably activated conditions.

15. Process according to claim 14, wherein said activated conditions are obtained by: (i) contacting said amine HNRₐR_{b} with a suitable activating agent such as trimethylaluminium, or (ii) converting said COOQ group into the corresponding acyl halide.

16. Use of a compound of formula (I) as defined in claims 1-9, or a pharmaceutically acceptable salt or hydrate thereof, in the preparation of a medicament for administration to a human or animal patient for modulating the activity of the ORL-1 receptors.

17. Use according to claim 16, wherein said drug is useful in the prophylaxis and treatment of illnesses dependent on modulation of the ORL-1 receptor.
